# EUROPEAN PATENT APPLICATION

(11) **EP 2 425 865 A1**
(43) Date of publication of application: **07.03.2012**
(21) Application number: 10172204.9
(22) Date of filing: 06.08.2010
(51) Int. Cl.: A61L 17/14

(54) **Medicinal thread having a polyhydroxyalkanoate coating**

(71) Applicant: Aesculap AG, 78532 Tuttlingen/Donau (DE)
(72) Inventor: Odermatt, Erich, Dr., Schaffhausen (CH); Bargon, Rainer, Dr., 78532 Tuttlingen (DE)
(74) Representative: Patentanwälte Ruff, Wilhelm, Beier, Dauster & Partner

(57) **Abstract**

The invention is related to a thread, comprising a thread body and a coating which at least partly, preferred completely, layers the thread body, wherein the coating comprises a polyhydroxyalkanoate comprising hydroxyalkanoic acid moieties having a chain of carbon atoms comprising at least four carbon atoms.

## Description

The invention is related to a thread, in particular a surgical thread, having a coating, comprising polyhydroxyalkanoates (PHAs), an implant, a surgical kit and a method for manufacturing the thread.

The function of a surgical suture material is predominantly to supply wound support needed in wound healing for a certain time period. To that end, the suture material is knotted using various tying methods and techniques. Thus, the knot of a suture material has essential importance to secure wound sealing and wound closure. As regards wound closures, the retention and securing capacity of a knot is significantly controlled by two determinants, namely knot fitting and knot holding (knot retention).

Herein, knot fitting is a measure for the load capacity of a knot subject to forces commonly resulting from movement of tissue or tissue layers. The forces may for example be strain on an anastomosis suture, as due to intestinal peristalsis, temporary strain on an aponeurosa suture of the abdominal wall caused by intermittent breathing, or strain on a blood vessel ligature caused by blood pulsating within the blood vessel, for example. With a bad knot fitting, there is a risk that all loops of the knot will slip open and the wound will tear up. Even if the loops do not slip open, there is a risk of wound dehiscence due to sliding of the tension thread.

On the other hand, knot holding or knot retention is a measure for the load capacity of a knot subject to external, in particular mechanical, impacts as may be caused in haemostasis of bleeding using a swab, for example. In case of bad knot retention, there may be twisting of thread ends and gradual release of knot loops resulting.

It is known that surface friction between thread ends, flexural stiffness of a thread as well as compressibility of a thread have a certain effect on knot fitting and knot retention (Tomita, N.; Tamai, S.; Morihara T.; lkeuchi K.; Ikada Y.: Handling Characteristics of Braided Suture Materials for Tight Tying, J. Appl. Biomat. 1993(4):61-65).

Further, it is also known that the tying technique employed has an effect on knot retention. It is noted, for example, that a positive effect on knot retention capacity of a thread is achieved by tension thread variation in the first instance and by tying of loops oriented in opposite directions in the second instance (Trimbos, JB; Van Rijseel, EJC; Klopper, PJ: Performance of Sliding Knots in Monofilament and Multifilament Suture Material. Obstet. Gyn. 1986(68):425-430).

Medical products prepared using poly-4-hydroxybutanoate are disclosed in patent publications US 2008/0095823 A1, EP 0 981 381 A2, and EP 1 659 142 A1, for example.

Yet, besides a secure knot fitting and knot retention, adequate handling characteristics of the suture material are also advantageous. Included therein are acceptable tissue passage performance, a smooth sliding surface to make knot loops slide along the tension thread, as well as a certain suppleness, flexibility, and compliance. There is always a certain risk to trade improvements in knot fitting and knot retention in for a loss of per se utile and desirable handling characteristics.

While in the case of monofilament suture materials approaches for improvement of knot fitting and knot retention are generally aimed at an appropriate choice of thread polymers (US 7,070,610 B2), in the case of multifilament suture materials there are usually coatings or braided structures employed to optimize knot fitting and knot retention of the suture materials.

EP 044 943 B1 discloses suture materials coated by carboxylic acid calcium salts, for example. EP 0 331 503 A2 describes suture materials having a coating of fatty acid esters. The fatty acid esters are usually carbohydrate fatty acid esters, like saccharose fatty acid esters, for example. However, such coating materials are in general not commercially available, and sometimes have to be prepared by means of extensive synthesis procedures. Furthermore, carbohydrate compounds are an ideal medium for microorganisms which in turn may be absorbed by multifilament suture materials via capillary forces. A coating of suture materials based on soft, viscous block copolymers is described in documents EP 0 261 470 A1 and EP 1 214 950 A1. A coating based on lactate containing copolymers for reinforcement of filaments is the object of EP 0 261 470 A1. Multi-component coatings, in particular based on surfactant type compounds, like fatty acid derivatives for example, and soft and/or reinforcing polymer components, are also disclosed in the prior art: US 5,716,373; US 4,201,216; US 5,609,609; WO 04/066927 A2, and US 2006/0188545 A1.

One disadvantage often encountered with the above mentioned approaches are the different dissolution characteristics of the involved components as well as the problems to ensure constant concentrations of all coating components during the coating procedure. To overcome the above drawbacks, sophisticated suspension coating processes (US 5,817,129) as well as water soluble coatings based on polyoxyalkylene glycols (US 5,312,473; US 4,047,533, and US 4,649,920) have been developed. One deficiency of water soluble polyoxyalkylene compounds is that during thread passage through a tissue, a significant portion of the coating will be stripped off even at the puncture site. Thus, the tissue passage behaviour of the thread will deteriorate as the thread is left partially uncoated. Furthermore, part of the coating may be stripped off as early as during ethylene oxide sterilization that is performed in a moist environment.

The present invention is based on the technical problem to provide a thread presenting improved thread characteristics, in particular in view of knot fitting, knot retention, knot run-down, performance during passage through tissue, and suppleness (flexibility, compliance).

The problem is solved according to the invention by a thread, in particular a surgical thread, comprising a thread body and a coating which at least partly, preferably completely, layers the thread body, wherein the coating comprises a polyhydroxyalkanoate. The polyhydroxyalkanoate comprises hydroxyalkanoic acid moieties (hydroxycarboxylic acid units) having a chain of carbon atoms comprising at least four carbon atoms.

In a preferred embodiment, the carbon chain includes from 4 to 15 carbon atoms, in particular 4 to 10 carbon atoms, preferably 4 to 8 carbon atoms, more preferably 4 to 6 carbon atoms.

It is preferred that the carbon atoms are directly linked to another in the carbon chain.

According to the invention, the hydroxyalkanoic acid moieties may in particular be hydroxyalkanoic acid moieties.

In a further embodiment, the hydroxyalkanoic acid moieties may include chirality centres. Thus, hydroxyalkanoic acid moieties may have chirality centres in an R- and/or S-configuration.

Preferably, the hydroxyalkanoic acid moieties are selected from the group consisting of 3-hydroxyalkanoic acid moieties, 4-hydroxyalkanoic acid moieties, 5-hydroxyalkanoic acid moieties, 6-hydroxyalkanoic acid moieties, enantiomers thereof, diastereomers thereof, and mixtures, in particular racemates, thereof.

It is preferred that the hydroxyalkanoic acid moieties are selected from the group consisting of 3-hydroxybutyrate, 4-hydroxybutyrate, 3-hydroxyvalerate, 4-hydroxyvalerate, 5-hydroxyvalerate, 3-hydroxyhexanoate, 4-hydroxyhexanoate, 5-hydroxyhexanoate, 6-hydroxyhexanoate, enantiomers thereof, diastereomers thereof, and mixtures, in particular racemates, thereof. Particularly preferred are 3-hydroxybutyrate, 4-hydroxybutyrate, 4-hydroxyvalerate, 5-hydroxyvalerate, enantiomers thereof, diastereomers thereof, and/or mixtures, in particular racemates, thereof.

In general, the polyhydroxyalkanoate may be a homopolymer or a copolymer. In terms of the present invention, a copolymer is to mean a polymer composed of at least two different monomer units, in particular at least two different hydroxyalkanoic acid moieties. Accordingly, the copolymer may also be a terpolymer, tetrapolymer or the like. In particular, the copolymer may be present as a random copolymer, block copolymer or segmented copolymer or graft copolymer. Furthermore, the copolymer may be an isotactic copolymer, syndiotactic copolymer or atactic copolymer.

In a further embodiment, the polyhydroxyalkanoate may be produced by synthesis or fermentation. Synthetic polyhydroxyalkanoates may be prepared based on monomeric lactones, and generally result in useful yields. The fermentative production of polyhydroxyalkanoates is well-known to a person skilled in the art from US 6,316,262, for example.

Further, the polyhydroxyalkanoate may include stereogenic centres or chirality centres. The latter may be present in an R- and/or S-configuration.

In another advantageous embodiment, the polyhydroxyalkanoate may be a poly-w-hydroxyalkanoic acid.

Preferably, the polyhydroxyalkanoate is selected from the group consisting of poly(3-hydroxyalkanoate), poly(4-hydroxyalkanoate), poly(5-hydroxyalkanoate), poly(6-hydroxyalkanoate), copolymers thereof and mixtures, in particular blends, thereof.

More preferably, the polyhydroxyalkanoate is selected from the group consisting of polyhydroxybutyrate, polyhydroxyvalerate, polyhydroxyhexanoate, copolymers thereof and combinations, in particular blends, thereof.

In an advanced embodiment, the polyhydroxyalkanoate is selected from the group consisting of poly(3-hydroxybutyrate), poly(4-hydroxybutyrate), poly(3-hydroxyvalerate), poly(4-hydroxyvalerate), poly(5-hydroxyvalerate), poly(3-hydroxyhexanoate), poly(4-hydroxyhexanoate), poly(5-hydroxyhexanoate), poly(6-hydroxyhexanoate), optical isomers thereof, copolymers thereof, and mixtures, in particular blends, thereof. Particularly preferred are poly(3-hydroxybutyrate), poly(4-hydroxybutyrate), poly(3-hydroxyvalerate), poly(5-hydroxyvalerate), optical isomers thereof, copolymers thereof, and mixtures, in particular blends, thereof. Particularly preferred copolymers are poly(4-hydroxybutyrate-co-3-hydroxybutyrate) and/or poly(5-hydroxyvalerate-co-3-hydroxyvalerate).

In another useful embodiment, the polyhydroxyalkanoate has a molecular mass from 1 to 1000 kDa (kilo Daltons), in particular from 400 to 1000 kDa, and preferred from 400 to 800 kDa.

The polyhydroxyalkanoate provided according to the invention, in particular poly(3-hydroxybutyrate) and/or poly(4-hydroxybutyrate), has the advantage that the hydroxyalkanoic acids resulting from hydrolytic degradation, in particular 3-hydroxybutyric acid and/or 4-hydroxybutyric acid, exhibit less acidity than glycolic acid and lactic acid, for example. Thus, the hydroxyalkanoic acid moieties included in the polyhydroxyalkanoate provided according to the invention will be released from the polymer at a later time, i. e., at a time of advanced wound healing, in particular after a first exsudative period. Thereby, hyperacidity of the wound area may be prevented during the first days following a surgical operation, where commonly inflammation responses are most predominant. In this way, the polyhydroxyalkanoate provided according to the invention may contribute essentially to tissue compatibility of the thread. Another advantage of the polyhydroxyalkanoate is that it may be degraded by means of enzymes, in particular within the body of a patient. During the enzymatic degradation, there is connective tissue encapsulation and with it a kind of "biological anchoring" of the suture. As a particularly beneficial effect, the anchoring provides for an early reinforcement of the incision site. Thus, the above mentioned tissue integration of the suture has a stabilizing effect on wound adaption.

Preferably, the coating is present in a proportion from 0.1 to 10 % by weight, in particular from 1 to 7 % by weight, and preferred from 2 to 5 % by weight, based on the total weight of the thread.

Further, the coating may have a layer thickness from 1 to 20 µm, in particular from 1 to 10 µm, preferred from 5 to 10 µm.

To improve knot fitting, the coating is with particular advantage provided such that it may absorb part of the mechanical strain acting on the thread within the body of a patient, without accumulating them in the form of energy, in particular elastic energy. In other words, the coating preferably has dissipative characteristics.

According to the invention, the coating may have a so-called loss modulus (viscous modulus) (G") from 5 to 50 MPa, in particular from 10 to 50 MPa, preferred from 10 to 30 MPa. In general, the loss modulus reflects the dissipative, i. e., irreversibly released viscous portion of the energy introduced into a system to be examined.

The thread body may have a loss modulus from 100 to 400 MPa, in particular from 200 to 300 MPa, preferred from 200 to 250 MPa.

The thread according to the invention may have a loss modulus from 30 to 60 MPa, in particular from 40 to 50 MPa, preferred from 45 to 50 MPa.

To improve knot fitting, the coating according to a further embodiment may have merely a low hardness. In particular, the coating exhibits a lower hardness than the thread body, whereby suppleness and compliance characteristics are imparted to the thread in total. A supple thread in turn advantageously permits improved knot run-down and improved passage through a tissue behaviour.

Preferably the coating has a hardness from 20 to 80 Shore D, in particular from 30 to 70 Shore D, preferred from 40 to 60 Shore D.

In a further advantageous embodiment, the coating is compressible, in particular elastically compressible. A particular advantage is that after tying down the knot twisting of thread ends and with it slippage and release of the knot is impeded by means of a compressible, in particular elastically compressible coating. A particularly beneficial effect thereby is an improvement in knot retention and holding of the thread. Another advantage of a compressible coating is that the thread is partially constricted during knot tying, whereby the frictional coefficient of the thread in the vicinity of the knot is increased and with it the knot security. The increase of the frictional coefficient in the vicinity of the knot may be attributed in particular to the formation of fine hairline cracks or fissures, in particular by forming "troughs" and "crests" on the coating surface, whereby slipping of the thread and with it coming undone of the knot may be impeded.

In an advanced embodiment, the coating is more elastic than the thread body. In this manner, the elasticity of the thread in total may be improved, whereby both the knot fitting and also the knot holding of the thread may be improved.

Preferably, the coating has a lower Young's modulus than the thread body. In particular the coating may have a Young's modulus < 1 GPa. More specifically, the coating may have a Young's modulus from 300 to 1000 MPa, particularly from 500 to 1000 MPa, preferably from 500 to 700 MPa, and more preferably from 600 to 700 MPa. The thread body may have a Young's modulus from 1 to 4 GPa, in particular from 2 to 3 GPa, preferably from 2.5 to 3 GPa. Particularly preferred is a Young's modulus of the thread body > 1 GPa.

The thread according to the invention preferably has a Young's modulus from 100 to 500 MPa, in particular from 200 to 400 MPa, preferred from 200 to 250 MPa.

In another advanced embodiment, the coating has a lower flexural modulus (bending modulus), i. e., flexural stiffness (bending stiffness) than the thread body. This also contributes to an improvement of knot fitting and knot retention of the thread. Preferably, the coating has a flexural modulus from 300 to 1000 MPa, in particular from 500 to 700 MPa, preferred from 600 to 700 MPa. Preferably, the thread body has a flexural modulus from 50 to 150 MPa, in particular from 50 to 100 MPa, preferred from 80 to 100 MPa.

The thread according to the invention preferably has a flexural modulus from 5 to 50 MPa, in particular from 20 to 40 MPa, preferred from 30 to 40 MPa.

In another preferred embodiment, the coating, in particular the polyhydroxyalkanoate, is at least partially crystalline or has crystalline regions at least in part thereof. Due to the presence of crystalline regions in the thread, in particular the knot run-down behaviour of the thread is improved.

Preferably, the coating, in particular the polyhydroxyalkanoate, has a melt enthalpy of more than 40 J/g, in particular more than 50 J/g, preferred more than 60 J/g.

In another advanced embodiment, the coating is in a solid state in a temperature range from 20 to 45 °C, in particular from 25 to 40 °C, preferred from 30 to 40 °C. In other words, with particular advantage the coating is not sticky, in particular not tenacious-viscous. In this way, undesirable adhesions to biological, in particular human and/or animal, tissue may be prevented, that otherwise could compromise the passage of the thread through tissue.

In another embodiment, the coating has a essentially, preferred completely, smooth surface. By means of a smooth coating surface, the passage behaviour of the thread through tissue may also be improved due to a reduced dynamic friction through the penetration hole into tissue. Another advantage of the smooth surface thread is that knot loops may exhibit superior run-down on a tension thread, for example. Additionally, threads having a smooth surface may have superior deformability, which is an advantage in particular during winding up of threads or during shipment and storage.

In a further advantageous embodiment, the thread has a diameter of in particular USP 2/0 gauge (corresponding to a diameter of 0.250 to 0.349 mm), and a knot breaking strength from 45 to 60 N, in particular from 45 to 55 N, preferred from 47 to 50 N. The knot breaking strength corresponds to the force, expressed in Newton (N), that is necessary to effect breaking of a knotted thread, wherein the force in the knot is measured. Here, the knot breaking strength is always less than the so-called linear tensile strength at break of a thread that corresponds to the force resulting in break of an unknotted or elongate thread. The linear tensile strength at break of the thread is preferably from 80 to 110 N, in particular from 80 to 90 N, preferred from 85 to 90 N.

In another embodiment, the thread, after 14 days of subjection to a Sörensen buffer, has a knot breaking strength corresponding to 65 to 70 %, in particular from 67 to 70 %, preferred from 68 to 70 % of the initial knot breaking strength, i. e., of the knot breaking strength measured prior to treatment in a Sörensen buffer. Preferably, after 21 days of subjection to a S6rensen buffer, the thread has a knot breaking strength corresponding to 30 to 40 %, in particular from 30 to 35 %, preferred from 30 to 33 % of the initial knot breaking strength of the thread.

The treatment of threads in a S6rensen buffer is a common method to simulate thread degradation "in vivo". To that end, the threads are typically immersed in an aqueous KH₂P0₄ and Na₂HP0₄ solution, with a pH value of 7.4 at 25 °C. The immersed threads are sampled from the buffer solution at predetermined time intervals and tested for linear tensile strength at break, knot breaking strength and the like, for example.

In general, the thread, in particular the coating may include additives, in particular active ingredients. The additives may be selected from the group consisting of antimicrobial, in particular antibiotic active agents, disinfecting active agents, antiphlogistic (anti-inflammatory) active agents, analgesic active agents, cell growth promoting active agents, cell recruiting active agents, cells and mixtures thereof.

In a particularly preferred embodiment, the coating is made essentially of polyhydroxyalkanoate. In this context, the expression "essentially" is to mean that the coating may include yet a small proportion of additional components, if need be, in particular additives like those mentioned in a preceding embodiment, for example, however, without impairing the advantageous characteristics of the thread according to the invention in a noteworthy amount. According to the invention, the coating may be provided to include a proportion of additional components of less than 1 % by weight, in particular less than 0.1 % by weight, preferred less than 0.01 % by weight, based on the total weight of the coating.

In an advanced embodiment, the coating is completely made of polyhydroxyalkanoate. In other words, the polyhydroxyalkanoate provided according to the invention is adequate as a coating component to impart beneficial characteristics to the thread, in particular in regard to knot fitting, knot tie down, knot retention, knot holding, knot run-down, knot slippage, passage through tissue behaviour, tissue drag, and the like.

The thread body as such may be absorbable, partially absorbable, or non-absorbable.

In general, the thread body is made of one or multiple polymers, in particular copolymers.

Preferably, the thread body is made of an absorbable polymer which is selected from the group consisting of polyglycolide, polylactide, poly-ε-caprolactone, trimethylene carbonate, poly-para-dioxanone, copolymers thereof, and combinations, in particular blends, thereof. A preferred copolymer is a copolymer comprising glycolide and lactide, in particular in a weight ratio from 9:1 to 1:9.

In particular, the thread body may be made of a polyhydroxyalkanoate, wherein the polyhydroxyalkanoate of the thread body differs from the polyhydroxyalkanoate of the coating, according to the invention.

In another embodiment, the thread body is made of a non-absorbable polymer which is selected from the group consisting of polyolefins such as polyethylene, polypropylene, polyvinylidene difluoride, polytetrafluoroethylene, in particular expanded polytetrafluoroethylene, polytetrafluoropropylene and/or polyhexafluoropropylene, for example, polyesters like polyethylene terephthalate, polypropylene terephthalate and/or polybutylene terephthalate, for example, polyurethanes, polyamides, such as nylon 6, nylon 6.6, for example, copolymers thereof, and combinations, in particular blends, thereof.

Preferably the thread body is made of polyethylene which is selected from the group consisting of low-density polyethylene (HDPE), high-density polyethylene (LDPE), high-molecular-weight polyethylene (HMWPE), ultra-high-molecular-weight polyethylene (UHMWPE), copolymers thereof, and mixtures, in particular blends, thereof. Particularly preferred is a thread body made of ultra-high-molecular-weight polyethylene (UHMWPE), in particular cross-linked ultra-high-molecular-weight polyethylene (UHMWPE).

In an advanced embodiment, the thread body is made of ultra high molecular weight polyethylene having an average molecular weight from 10⁴ to 10⁷ g/mol, in particular from 10⁵ to 10⁶ g/mol.

The thread according to the invention may in principle be a monofilament, pseudomono-filament or multifilament thread. Preferably, the thread is a multifilament, in particular a braided or twisted thread. In particular, individual fibres or filaments of a multifilament thread may each be coated, in particular each be completely coated.

Furthermore, the thread may have a core-sheath structure, wherein the core is represented by the thread body and the sheath is represented by the coating. The thread may in particular be prepared as a coextrusion filament or as a sheath extrusion filament.

In another embodiment, the thread is provided as a sterilized thread, for example by ethylene oxide gassing, und in particular in a finished, cut-to-length package.

In a particularly preferred embodiment, the thread is provided as a surgical suture material.

In another embodiment, the thread is connected to one or multiple surgical needles.

The present invention further relates to an implant, particularly a surgical implant, comprising at least one thread including a thread body and a coating which at least partly, preferably completely, layers the thread body. The coating comprises a polyhydroxyalkanoate comprising hydroxyalkanoic acid moieties having a chain of carbon atoms comprising at least four carbon atoms.

In a preferred embodiment, the implant is a surgical suture material. On the other hand, the implant may be another surgical textile, in particular a surgical mesh or surgical pad, like a hernia mesh, prolapse mesh or incontinence mesh, according to the invention. As regards further features and advantages of the implant according to the invention, in particular in relation to the thread, reference is made to the complete description mentioned above.

The present invention further encompasses a surgical kit, comprising at least one, in particular one or multiple, i. e. at least two, surgical needle(s) and a thread or implant according to the invention. As regards further features and advantages of the kit, in particular in relation to the thread or the implant, reference is made to the complete description mentioned above.

Furthermore, the present invention is related to a method for manufacturing a thread, wherein a thread body is coated with a polyhydroxyalkanoate comprising hydroxyalkanoic acid moieties having a chain of carbon atoms comprising at least four carbon atoms.

In an advantageous embodiment, the coating may be applied by means of core-sheath extrusion. In general, the procedure may be performed by coextrusion of the thread body and the polyhydroxyalkanoate. A core-sheath structure of the thread may be realized by bicomponent extrusion, for example.

In an alternative embodiment, the thread body may be coated by means of sheath extrusion of the polyhydroxyalkanoate. In this embodiment, the thread body may be a monofilament or a multifilament, in particular a braided or twisted multifilament. The use of a multifilament thread body for the sheath extrusion results in a pseudomonofilament thread. The sheath extrusion is in particular appropriate in coating of high melting thread bodies, like thread bodies made of glycolide, lactide or copolymers thereof, using low melting polyhydroxyalkanoate, like poly(4-hydroxybutyrate), for example.

In a further embodiment, the coating is applied by means of an impregnation, wetting, dipping, immersion, spraying, brushing, roller and/or calendering coating technique. Depending on the respective coating technique employed, it may be convenient to use the polyhydroxyalkanoate in a liquid dispersion, suspension, solution or molten form.

The above described techniques for coating the thread body are relatively simple and in particular cost-efficient to employ, and furthermore allow both a partial coating and a complete, i. e. all over the surface, coating of the thread body.

In a preferred embodiment, a solution coating procedure is performed to provide the thread body with a polyhydroxyalkanoate coating. To that end, preference is given to a solution of the polyhydroxyalkanoate including an amount of the polyhydroxyalkanoate from 2 to 10 % by weight, in particular from 3 to 7 % by weight, preferred from 2 to 5 % by weight, based on the total weight of the solution. An adequate solvent may be cyclopentanone, for example. Cyclopentanone is beneficial in that it is a non-toxic, hardly flammable, and ecologically (as to environmental compatibility aspects) acceptable solvent. For solution coating, the thread body is preferably immersed or dipped into a solution of the polyhydroxyalkanoate or sprayed with a solution thereof.

As to further features and advantages of the method according to the invention, reference is made to the complete above description of the thread according to the invention.

Besides, the present invention is related to a method for manufacturing an implant, wherein at least one thread body is coated with a polyhydroxyalkanoate comprising hydroxyalkanoic acid moieties having a chain of carbon atoms comprising at least four carbon atoms. As to further features and advantages of the method according to the invention, reference is made to the complete above description of the thread according to the invention.

Finally, the invention is also related to use of a polyhydroxyalkanoate comprising hydroxyalkanoic acid moieties having a chain of carbon atoms comprising at least four carbon atoms for the production of a thread or implant according to the invention. As to further features and advantages, in particular in regard to the polyhydroxyalkanoate as well as the thread as such, reference is made to the complete above description.

Below, the advantages of the invention will be summarized again as follows:
The thread according to the invention presents improved knot characteristics, in particular in view of knot fitting, knot retention, and knot run-down behaviour, due to a particular polyhydroxyalkanoate coating. In this manner, the security of knots may be improved and a risk of wound dehiscence may be minimized significantly.

Another advantage is related to an improved degradation characteristic of the thread. Thus, the hydroxyalkanoic acids produced during degradation of the polyhydroxyalkanoate provided according to the invention, in particular 3-hydroxybutyric acid and/or 4-hydroxybutyric acid as released from poly(3-hydroxybutyrate) and/or poly(4-hydroxybutyrate), have a minor acidity, whereby postoperative hyperacidity of the wound environment, and thus undesired enhancement of inflammation responses may be prevented, in particular during the initial wound healing period.

Yet another advantage is related to excellent biological anchoring of the thread. Thus, there is fibrous connective tissue encapsulation in the vicinity of the incision site even at an early time post surgery, whereby tissue integration of the thread and thus security of wound sealing and wound closure are improved.

For development of the beneficial characteristics of the thread, it would be sufficient that the coating includes merely the polyhydroxyalkanoate provided according to the invention as a coating component. Thus, further components, as for example those well-known in the state of the art, are unnecessary. As a particular advantage, this allows for a simple and in particular cost-efficient technical realization of the thread according to the invention.

Further features and advantages of the present invention will be apparent from the following description of preferred embodiments illustrated for examples and in connection with features of the dependent claims. Therein, individual features may be embodied alone or in combination of two or more.

### Example 1: Coating of a polyglycolide thread using poly(4-hydroxybutyrate)

A polyglycolide thread of USP 2/0 gauge was pulled through a cyclopentanone solution including an amount of 4 % by weight of poly(4-hydroxybutyrate), based on the total weight of the solution, at a rate of 5 m/min in a coating device. Then, the thread was dried in a heating duct at 150 °C over a length of 2 m and wound up. The polymer content on the thread was about 2.24 % by weight, based on the total weight of the thread.

Subsequently, the thread was subject to a degradation test in Sörensen buffer. Knot breaking strength or knot breaking load were determined at day 0, 14 and 21, according to EP directives. The values obtained therein are listed below in table 1.

**Table 1**

| Degradation in Sörensen buffer | Day 0 | Day 14 | Day 21 |
|---|---|---|---|
| Knot breaking load | 47.6 N | 32.6 N | 15.7 N |
| Breaking load retention [%] | - | 69 | 33 |
| Elongation [%] | 22 % | 11 % | 6 % |

### Example 2: Evaluation of knot fitting with a polyglycolide thread coated according to example 1

Six tied down loops were placed around six pulsating cylinders made of synthetic material and provided with a silicone coating, and subject to cyclic loading for 1000 cycles. A total of two runs each using six knots were carried out. The knot combinations used were slip knots (running knots) of the S x S x S nomenclature, in all cases. The load force applied to the interior of the loops through the cylinders was about 1.5 N. After termination of the experiment all the 12 knots proved to be closed.

### Example 3: Evaluation of bending stiffness modulus with a polyglycolide thread coated according to example 1

A polyglycolide thread section provided with a poly(4-hydroxybutyrate) coating in a length of 1 mm and USP 2/0 gauge, was subject to a bending test in a Frank bending stiffness tester 58565 at a test rate of 6°/s, a bending angle to 30°, a holding time of 2 s, and a preload of 1 mN. The polyglycolide thread exhibited a bending module of 92 MPa. A Vicryl® thread of USP 2/0 gauge as supplied by the Ethicon company was considered as a commercial standard for comparison. The Vicryl® thread exhibited a bending module of 130 MPa.

### Example 4: Evaluation of knot run-down (surface roughness)

Frictional force differences occurring during sliding of two coated Safil® samples according to example 1 one against the other were tested in a knot run-down test apparatus. To that end, the test on a knot run-down board was conducted using a Zwick & Roell Z005 Allround Tabletop Tester equipped with a 100 N load cell and a Z6FD1 weighing cell. Two coated threads according to example 1 were twisted twice with each other, led around pulleys of the tester, and compressed in the upper clamping jaw. A weight (2 x 98.1 g) was attached to each of the thread ends and the cross bar lifted at a rate of 400 mm/min. Subsequently, the standard deviation of the evaluated forces was recorded. The obtained force deviation was 0.19 N for the coated thread according to example 1 and 0.20 N for the comparative Vicryl® thread of the same gauge. The uncoated Safil® thread exhibited a standard deviation of 0.24 N.

### Example 5: Evaluation of Young's modulus

The ratio of loss modulus to Young's modulus was evaluated in line with a dynamic-mechanical analysis. The initial tension was set to 1 Newton and measurement was conducted in a temperature-sweep mode at a heating rate of 2 °K/min starting from 25 °C up to 180 °C. A dynamic elongation of 50 µm was selected. The suture material according to example 1 exhibited a tan δ of 0.0843. A Vicryl® thread used for comparison exhibited a tan 6 of 0.0668. Furthermore, the glass transition temperatures were evaluated for the thread according to example 1. The glass transition temperature of the thread according to example 1 was found to be 71.5 °C. The Vicryl® thread exhibited a glass transition temperature of 73.5 °C.

## Claims

1. A thread, in particular a surgical thread, comprising a thread body and a coating which at least partly, preferably completely, layers the thread body, **characterized in that** the coating comprises a polyhydroxyalkanoate comprising hydroxyalkanoic acid moieties having a chain of carbon atoms comprising at least four carbon atoms.

2. The thread according to claim 1, **characterized in that** the chain of carbon atoms comprises 4 to 15 carbon atoms, in particular 4 to 10 carbon atoms, preferably 4 to 8 carbon atoms, more preferably 4 to 6 carbon atoms.

3. The thread according to claim 1 or 2, **characterized in that** the polyhydroxyalkanoate is selected from the group consisting of poly(3-hydroxyalkanoate), poly(4-hydroxyalkanoate), poly(5-hydroxyalkanoate), poly(6-hydroxyalkanoate), copolymers thereof and mixtures, in particular blends, thereof.

4. The thread according to one of the preceding claims, **characterized in that** the polyhydroxyalkanoate is selected from the group consisting of polyhydroxybutyrate, polyhydroxyvalerate, polyhydroxyhexanoate, copolymers thereof and combinations, in particular blends, thereof.

5. The thread according to one of the preceding claims, **characterized in that** the polyhydroxyalkanoate is selected from the group consisting of poly(3-hydroxybutyrate), poly(4-hydroxybutyrate), poly(3-hydroxyvalerate), poly(4-hydroxyvalerate), poly(5-hydroxyvalerate), poly(3-hydroxyhexanoate), poly(4-hydroxyhexanoate), poly(5-hydroxyhexanoate), poly(6-hydroxyhexanoate), copolymers thereof, optical isomers thereof, and combinations, in particular blends, thereof.

6. The thread according to one of the preceding claims, **characterized in that** the coating is present in a proportion from 0.1 to 10 % by weight, in particular from 1 to 7 % by weight, and preferred from 2 to 5 % by weight, relating to the total weight of the thread.

7. The thread according to one of the preceding claims, **characterized in that** the coating has a layer thickness from 1 to 20 µm, in particular from 1 to 10 µm, preferred from 5 to 10 µm.

8. The thread according to one of the preceding claims, **characterized in that** the coating has a loss modulus (viscous modulus) from 5 to 50 MPa, in particular from 10 to 50 MPa, preferred from 10 to 30 MPa.

9. The thread according to one of the preceding claims, **characterized in that** the coating has a hardness from 20 to 80 Shore D, in particular from 30 to 70 Shore D, preferred from 40 to 60 Shore D.

10. The thread according to one of the preceding claims, **characterized in that** the coating has a Young's modulus from 300 to 1000 MPa, preferably from 500 to 700 MPa, and more preferably from 600 to 700 MPa.

11. The thread according to one of the preceding claims, **characterized in that** the thread body is made of an absorbable polymer, in particular a polyhydroxyalkanoate that is different to the polyhydroxyalkanoate of the coating, preferably selected from the group consisting of polyglycolide, polylactide, poly-s-caprolactone, trimethylencarbonate, poly-para-dioxanone, copolymers thereof and combinations, in particular blends, thereof.

12. The thread according to one of the claims 1 to 10, **characterized in that** the thread body is made of a non absorbable polymer, in particular selected from the group consisting of polyolefins such as polyethylene, polypropylene, polyvinylidene difluoride, polytetrafluoroethylene, in particular expanded polytetrafluoroethylene, polytetrafluoropropylene or polyhexafluoropropylene, polyesters such as polyethylene terephthalate, polypropylene terephthalate or polybutylene terephthalate, polyurethane, polyamide such as nylon 6.6 or nylon 6, copolymers thereof and combinations, in particular blends, thereof.

13. The thread according to one of the preceding claims, **characterized in that** the thread is designed as a surgical suture.

14. A surgical implant, in particular a surgical suture, comprising at least one thread according to one of the preceding claims.

15. A surgical kit comprising at least one surgical needle and a thread according to one of the claims 1 to 13 or a surgical implant according to claim 14.

16. A method for manufacturing a thread, in particular according to one of the claims 1 to 13, wherein a thread body is coated with a polyhydroxyalkanoate comprising hydroxyalkanoic acid moieties having a chain of carbon atoms comprising at least four carbon atoms.
